# EUROPEAN PATENT APPLICATION

(11) **EP 4 390 191 A1**
(43) Date of publication of application: **26.06.2024**
(21) Application number: 23758928.8
(22) Date of filing: 16.01.2023
(51) Int. Cl.: F16K 11/02

(54) **GAS-LIQUID SWITCHING VALVE AND ENDOSCOPE**

(30) Priority: 24.02.2022 CN 202210169121
(71) Applicant: Hangzhou Leinzett Medical Technology Co., Ltd., Hangzhou, Zhejiang 311115 (CN)
(72) Inventor: ZHANG, Jian, Hangzhou, Zhejiang 311115 (CN); LIU, Menghua, Hangzhou, Zhejiang 311115 (CN); SHEN, Heliang, Hangzhou, Zhejiang 311115 (CN); WAN, Jixian, Hangzhou, Zhejiang 311115 (CN)
(74) Representative: Nederlandsch Octrooibureau
(86) International application number: PCT/CN2023/072299
(87) International publication number: WO 2023/160303

(57) **Abstract**

The present application provides a gas-liquid switching valve and an endoscope, which can prevent liquid from flowing out of the switching valve, contributing to improving its safety performance. The gas-liquid switching valve includes a valve body, a valve core assembly, and a liquid-proof and gas-permeable member. The valve core assembly is movably disposed in a valve cavity, and the valve core assembly is provided with a communication channel for communicating with an external environment. When the valve core assembly is moved to a first position, a liquid path between a liquid inlet and a gas-liquid outlet is blocked, and a gas path between a gas inlet and the gas-liquid outlet is unblocked, and at this time, the communication channel is blocked so that the gas-liquid switching valve is in a gas supply state. When the valve core assembly is moved to a second position, the gas path between the gas inlet and the gas-liquid outlet is blocked, and the liquid path between the liquid inlet and the gas-liquid outlet is unblocked, so that the gas-liquid switching valve is in a liquid supply state. The liquid-proof and gas-permeable member is disposed in the communication channel, and is configured to prevent liquid from passing through the communication channel and allow gas to pass through the communication channel.

## Description

### TECHNICAL FIELD

The present application relates to the technical field of endoscopes, in particular to a gas-liquid switching valve and an endoscope.

### BACKGROUND

In the medical field and the industrial field, endoscopes having an elongated insertion portion which can be inserted into a body cavity or a duct are widely used, and are generally provided with air and liquid supply devices, which can supply water, liquid medicine, air, and the like to the body cavity or the duct as needed.

Currently, gas/liquid supply for an endoscope is controlled through a gas-liquid switching valve disposed in an operation portion of the endoscope, and in order to achieve switching of three states of gas supply, liquid supply, and neither gas supply nor liquid supply for the endoscope, a natural state of a switching valve employed in the existing endoscope is generally that a gas channel communicates with the outside atmosphere through a through hole at a top of the switching valve, and a liquid channel is closed by a valve core of the switching valve, so that neither gas supply nor liquid supply is achieved. Thereafter, if the top of the switching valve is pressed so that the liquid channel is opened, the endoscope is in a liquid supply state; and if the through hole at the top of the switching valve is blocked to disconnect the gas channel from the outside atmosphere, the endoscope is in a gas supply state.

In particular, an internal structure of a gas-liquid switching valve of an endoscope in an early stage is very complicated, and parts were numerous, so that the cost of both use and maintenance is very high. In order to solve this problem, an existing Chinese invention patent CN110522396A provides a medical endoscope and a water-gas switching valve thereof, wherein the water-gas switching valve includes a valve body, and a gas inlet, a gas outlet, a liquid inlet, and a liquid outlet which are formed in a side wall of the valve body along a length direction of the valve body, and a valve core slidably disposed in the valve body along the length direction of the valve body; when the valve core slides to a first state position, the gas inlet communicates with the gas outlet, and the liquid inlet and the liquid outlet are closed, and when the valve core slides to a second state position, the gas inlet and the gas outlet are closed, and the liquid inlet communicates with the liquid outlet. Another existing Chinese invention patent CN104224090A provides a fluid switching valve for an endoscope, including a valve cover, a valve rod, a valve core, an elastic member, a button, a first sealing ring, a second sealing ring, a third sealing ring, a fourth sealing ring, a gas inlet, a liquid inlet, and a fluid outlet, so that the structure of the fluid switching valve for an endoscope is simpler by providing only one fluid outlet, helping to save the internal space.

However, when the top of the switching valve is pressed to switch the endoscope the liquid supply state, at the moment when the liquid channel is opened, there may be liquid flushing into the through hole at the top of the switching valve, and then flowing out of the through hole, which will not only wet or contaminate the surface of the switching valve, but also affects a circuit board or other electrical parts in the operation portion, thereby affecting the normal use of the endoscope, and once the liquid flushing out flows back into the gas channel of the switching valve upon contact with the external environment (e.g., a user's finger, etc.), the contaminated backflow liquid is fed into the body cavity when the endoscope is switched to the gas supply state, easily causing infection, and causing a great challenge to the safety of the endoscope.

### SUMMARY

An advantage of the present application is to provide a gas-liquid switching valve and an endoscope, which can prevent liquid from flowing out of the switching valve, contributing to improving its safety performance.

Another advantage of the present application is to provide a gas-liquid switching valve and an endoscope, wherein in one embodiment of the present application, the gas-liquid switching valve can prevent liquid from passing through a communication channel while allowing gas to pass through the communication channel through a liquid-proof and gas-permeable member, so as to effectively solve the problem of liquid flushing out occurring when the switching valve is operated.

Another advantage of the present application is to provide a gas-liquid switching valve and an endoscope, wherein in one embodiment of the present application, the gas-liquid switching valve can be simplified in structure, easily disassembled and assembled, easily cleaned, and sterilized.

Another advantage of the present application is to provide a gas-liquid switching valve and an endoscope, wherein in one embodiment of the present application, the gas-liquid switching valve can maintain a gas pressure at a gas outlet in a natural state to prevent a body fluid from flowing backward.

Another advantage of the present application is to provide a gas-liquid switching valve and an endoscope, wherein no expensive materials or complicated structures need to be used in the present application in order to achieve the above advantages. Thus, the present application successfully and effectively provides a solution that not only provides a simple gas-liquid switching valve and an endoscope, but also increases the practicality and reliability of the gas-liquid switching valve and the endoscope.

Based on this, in order to achieve at least one of the above advantages or other advantages and objects of the present application, the present application provides a gas-liquid switching valve, including:
a valve body provided with a valve cavity, a gas inlet, a liquid inlet, and a gas-liquid outlet, wherein the gas inlet, the liquid inlet and the gas-liquid outlet all communicate with the valve cavity;
a valve core assembly, wherein the valve core assembly is movably disposed in the valve cavity to move between a first position and a second position, and the valve core assembly is provided with a communication channel for communicating the valve cavity with an external environment, wherein when the valve core assembly is moved to the first position, a liquid path between the liquid inlet and the gas-liquid outlet is blocked by the valve core assembly, and a gas path between the gas inlet and the gas-liquid outlet is unblocked, and at this time, the communication channel is blocked so that the gas-liquid switching valve is in a gas supply state; wherein when the valve core assembly is moved to the second position, a gas path between the gas inlet and the gas-liquid outlet is blocked by the valve core assembly, and a liquid path between the liquid inlet and the gas-liquid outlet is unblocked, so that the gas-liquid switching valve is in a liquid supply state; and
a liquid-proof and gas-permeable member, wherein the liquid-proof and gas-permeable member is disposed in the communication channel of the valve core assembly, and configured to prevent liquid from passing through the communication channel of the valve core assembly, and allow gas to pass through the communication channel of the valve core assembly.

According to one embodiment of the present application, the liquid-proof and gas-permeable member includes a waterproof gas-permeable membrane, and the waterproof gas-permeable membrane is disposed at an outlet of the communication channel of the valve core assembly.

According to one embodiment of the present application, the liquid-proof and gas-permeable member further includes a hollow substrate, wherein the hollow substrate is fixed to the valve core assembly, and the waterproof gas-permeable membrane is supportively attached to the hollow substrate.

According to one embodiment of the present application, the valve core assembly includes a valve core shaft, a first sealing element configured to unblock and block a gas path between the gas inlet and the gas-liquid outlet, and a second sealing element configured to unblock and block a liquid path between the liquid inlet and the gas-liquid outlet, and the valve core shaft is provided with a valve core cavity having an opening, and a communication hole located at a side wall of the valve core cavity to form the communication channel of the valve core assembly through the valve core cavity and the communication hole, wherein the valve core shaft is axially slidably disposed in the valve cavity of the valve body, and the first sealing element and the second sealing element are spacedly disposed on the valve core shaft.

According to one embodiment of the present application, the valve cavity of the valve body is provided with a first sealing face located between the gas inlet and the gas-liquid outlet, and a second sealing face located between the liquid inlet and the gas-liquid outlet, wherein the first sealing element airtightly abuts against the first sealing face when the valve core shaft of the valve core assembly is pressed to move to the second position, and the second sealing element airtightly abuts against the second sealing face when the valve core shaft of the valve core assembly is lifted to move to the first position.

According to one embodiment of the present application, the valve body includes an annular sealing platform protruding from a side wall of the valve cavity to respectively provide the first sealing face and the second sealing face by upper and lower end surfaces of the annular sealing platform.

According to one embodiment of the present application, the gas-liquid switching valve further includes a reset mechanism, wherein the reset mechanism is disposed on the valve body, and is configured to apply a reset force to the valve core shaft so that the valve core shaft is automatically lifted to return to the first position after a pressing force is removed.

According to one embodiment of the present application, the reset mechanism includes a control member and an elastic member, wherein the control member is fixedly connected to the valve core shaft, and is partially exposed outside the valve cavity of the valve body, wherein the elastic member is correspondingly disposed between the control member and the valve body, and is configured to apply an elastic force away from the valve body to the control member.

According to one embodiment of the present application, the control member is a button, and the elastic member is a spring, wherein the button is detachably connected to the valve core shaft, and one end of the spring abuts against the button, and the other end of the spring abuts against the valve body.

According to one embodiment of the present application, the valve core shaft has a sealing end, and a control end extending axially from the sealing end, wherein the first sealing element and the second sealing element spacedly sleeve the sealing end of the valve core shaft, and the valve core cavity is located at the control end of the valve core shaft, wherein a through hole, and an annular cavity surrounding the through hole are formed in the button, the control end of the valve core shaft is fixedly inserted into the through hole of the button for communicating the opening of the valve core cavity with an external environment of the valve cavity through the through hole, and the spring is sleeved with the annular cavity of the button.

According to one embodiment of the present application, the through hole of the button is provided with convex threads.

According to one embodiment of the present application, the valve core assembly further includes a third sealing element, wherein the third sealing element is located between the button and the valve body to hermetically block a gap between the button and the valve body.

According to one embodiment of the present application, the sealing end of the valve core shaft is provided with a first mounting groove close to the control end, and a second mounting groove away from the control end, and the first sealing element is embedded into the first mounting groove, and the second sealing element is embedded into the second mounting groove, wherein an inner diameter dimension of the valve cavity of the valve body at an outlet cavity is greater than a maximum radial dimension of the first mounting groove at the sealing end, and is less than a maximum radial dimension of the second mounting groove at the sealing end.

According to one embodiment of the present application, the sealing end of the valve core shaft is further provided with a support platform for supporting the second sealing element, and a radial dimension of the support platform is greater than an inner diameter dimension of the annular sealing platform.

According to one embodiment of the present application, the valve body includes a valve cylinder body having a hollow structure penetrating from top to bottom, and a valve cover body, wherein the valve cover body is detachably mounted at a lower end of the valve cylinder body, and the control member of the reset mechanism is correspondingly disposed at an upper end of the valve cylinder body.

According to another aspect of the present application, an embodiment of the present application further provides an endoscope, including:
an endoscope body; and
any one gas-liquid switching valve described above, wherein the gas-liquid switching valve is correspondingly disposed in an operation portion of the endoscope body for switching gas and liquid supplied to an insertion portion of the endoscope body.

### BRIEF DESCRIPTION OF THE DRAWINGS

In order to illustrate the technical solutions in the embodiments of the present application or the conventional technology more clearly, the accompanying drawings required to be used in the description of the embodiments or the conventional technology will be simply introduced below, obviously, the accompanying drawings in the following description are only some embodiments of the present application, and for those of ordinary skill in the art, other drawings can also be obtained according to these drawings without paying inventive steps.
FIG. 1 is a schematic perspective view of a gas-liquid switching valve according to an embodiment of the present application;
FIG. 2 shows an exploded schematic view of the gas-liquid switching valve according to the above embodiment of the present application;
FIG. 3 shows a cross-sectional schematic view of the gas-liquid switching valve in a natural state according to the above embodiment of the present application;
FIG. 4 shows a cross-sectional schematic view of the gas-liquid switching valve in a gas supply state according to the above embodiment of the present application;
FIG. 5 shows a cross-sectional schematic view of the gas-liquid switching valve in a liquid supply state according to the above embodiment of the present application;
FIG. 6 is a structural schematic diagram of an endoscope according to an embodiment of the present application.

Reference signs: 1. gas-liquid switching valve; 10. valve body; 100. valve cavity; 101. gas inlet; 102. liquid inlet; 103. gas-liquid outlet; 110. annular sealing platform; 1101. first sealing face; 1102. second sealing face; 11. valve cylinder body; 12. valve cover body; 20. valve core assembly; 200. communication channel; 21. valve core shaft; 201. valve core cavity; 202. communication hole; 211. sealing end; 2111. first mounting groove; 2112. second mounting groove; 2113. support platform; 212. control end; 22. first sealing element; 23. second sealing element; 24. third sealing element; 30. liquid-proof and gas-permeable member; 31. waterproof gas-permeable membrane; 32. hollow substrate; 40. reset mechanism; 41. control member; 410. button; 411. through hole; 412. annular cavity; 413. convex thread; 42. elastic member; 420. spring; and 2. endoscope body.

### DETAILED DESCRIPTION

In order to make the above objects, features and advantages of the present application more obvious and understandable, specific embodiments of the present application will be described below in detail with reference to the accompanying drawings. Numerous specific details are set forth in the following description in order to provide a thorough understanding of the present application. However, the present application may be implemented in many ways different from those described herein, and those skilled in the art may make similar modifications without departing from the connotation of the present application. Therefore, the present application is not limited by the specific embodiments disclosed below.

It should be noted that when a component is referred to as being "fixed to" or "disposed on" another component, it can be directly on another component or an intermediate component may also be present. When a component is referred to as being "connected" to another component, it can be directly connected to another component or an intermediate component may also be present. The terms "vertical", "horizontal", "upper", "lower", "left", "right", and the like used in the description of the present application are for illustrative purposes only and do not denote the only embodiment.

In addition, the terms "first" and "second" are used for descriptive purposes only and cannot be understood to indicate or imply relative importance or to implicitly specify the number of technical features indicated. Thus, features defined as "first" and "second" may explicitly or implicitly include at least one of the features. In the description of the present application, "a plurality of means at least two, e.g., two, three, etc., unless expressly and specifically defined otherwise.

In the present application, unless expressly specified and defined otherwise, a first feature is "on" and "under" a second feature, which may be that the first feature is directly in contact with the second feature, or the first feature and the second feature are indirectly in contact through an intermediate medium. Moreover, a first feature is "on", "above", and "over" a second feature, which may be that the first feature is directly above or obliquely above the second feature, or simply mean that a horizontal height of the first feature is higher than that of the second feature. A first feature is "under", "below", and "beneath" a second feature, which may be that the first feature is directly below or obliquely below the second feature, or simply mean that a horizontal height of the first feature is less than that of the second feature.

Unless defined otherwise, all technical and scientific terms used in the description of the present application have the same meaning as commonly understood by those skilled in the art to which the present application belongs. The terms used in the description of the present application are for the purpose of describing specific embodiments only, and are not intended to be limiting of the present application. The term "and/or" used in the description of the present application includes any and all combinations of one or more of the associated listed items.

Considering that at the moment when a switching valve employed in the existing endoscope is pressed to open a liquid channel, there may be liquid flushing into a through hole at a top of the switching valve to flow out of the through hole, thereby affecting the normal use and safety of the endoscope, the present application provides a gas-liquid switching valve and an endoscope, which can prevent liquid from passing through a communication channel while allowing gas to pass through the communication channel to effectively solve the problem of liquid flushing out occurring when the switching valve is operated. In addition, after exploring the reasons why the above problem occurs, it is found that a gas path and a liquid path are overlapped in space due to the limitation of the structure in general, and liquid remains in the overlapped space so as to enter the gas path when the gas path is opened, and the present application solves the above problem.

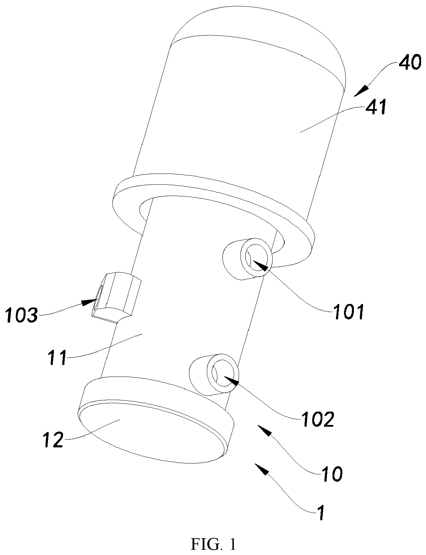

Specifically, referring to FIGS. 1 to 5, an embodiment of the present application provides a gas-liquid switching valve 1, which may include a valve body 10, a valve core assembly 20, and a liquid-proof and gas-permeable member 30. The valve body 10 may be provided with a valve cavity 100, a gas inlet 101, a liquid inlet 102, and a gas-liquid outlet 103, wherein the gas inlet 101, the liquid inlet 102 and the gas-liquid outlet 103 all communicate with the valve cavity 100. The valve core assembly 20 is movably disposed in the valve cavity 100 to move between a first position and a second position, and the valve core assembly 20 is provided with a communication channel 200 for communicating the valve cavity 100 with an external environment, wherein when the valve core assembly 20 is moved to the first position, a liquid path between the liquid inlet 102 and the gas-liquid outlet 103 is blocked by the valve core assembly 20, a gas path between the gas inlet 101 and the gas-liquid outlet 103 is unblocked, and at this time, the communication channel 200 is blocked so that the gas-liquid switching valve 1 is in a gas supply state; wherein when the valve core assembly 20 is moved to the second position, a gas path between the gas inlet 101 and the gas-liquid outlet 103 is blocked by the valve core assembly, and a liquid path between the liquid inlet 102 and the gas-liquid outlet 103 is unblocked, so that the gas-liquid switching valve 1 is in a liquid supply state. The liquid-proof and gas-permeable member 30 is disposed in the communication channel 200 of the valve core assembly 20, and configured to prevent liquid from passing through the communication channel 200 of the valve core assembly 20, and allow gas to pass through the communication channel 200 of the valve core assembly 20.

Notably, it is precisely because the liquid-proof and gas-permeable member 30 is disposed in the communication channel 200 of the valve core assembly 20, so that the communication channel 200 only allows gas to pass through, but does not allow liquid to pass through, even if there is liquid flushing into the communication channel 200 at the moment of pressing the valve core assembly 20, the liquid will not flush out of the communication channel 200 under the blocking of the liquid-proof and gas-permeable member 30, preventing the liquid from contaminating the switching valve or the liquid from being contaminated, helping to ensure the normal use and safety of the switching valve.

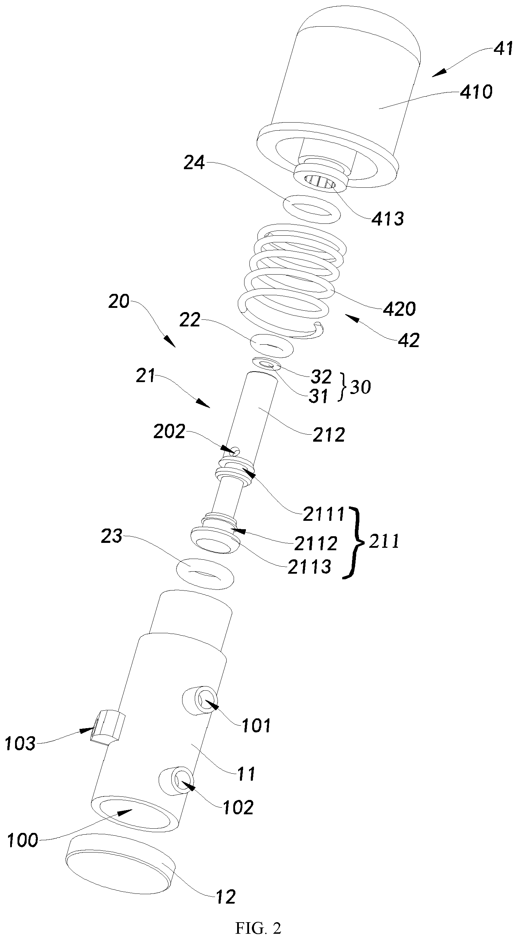

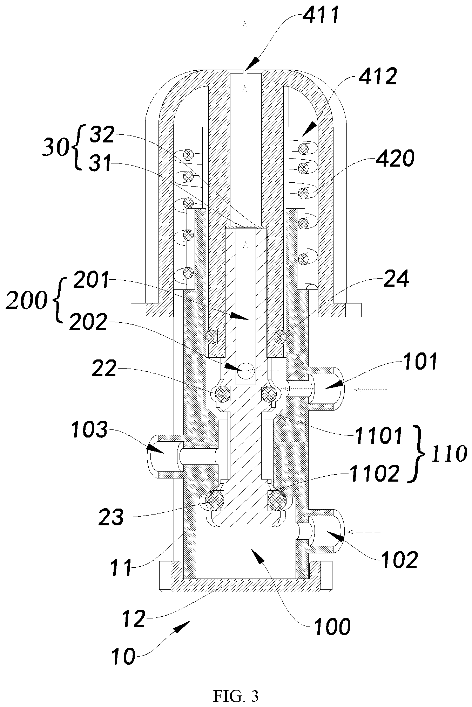

More specifically, as shown in FIGS. 2 and 3, the liquid-proof and gas-permeable member 30 of the present application may include, but is not limited to, a waterproof gas-permeable membrane 31 for allowing gas to pass through while preventing water from passing through, and the waterproof gas-permeable membrane 31 is disposed at an outlet of the communication channel 200 in order to install, replace or maintain the waterproof gas-permeable membrane 31.

Optionally, as shown in FIGS. 2 and 3, the liquid-proof and gas-permeable member 30 may further include a hollow substrate 32, wherein the hollow substrate 32 is fixed to the valve core assembly 20, and the waterproof gas-permeable membrane 31 is attached to the hollow substrate 32 to support the waterproof gas-permeable membrane 31 by the hollow substrate 32, enhancing the structural stability of the waterproof gas-permeable membrane 31, and reducing the risk that the waterproof gas-permeable membrane 31 is deformed or broken by airflow or liquid.

According to the above embodiment of the present application, the gas inlet 101 in the valve body 10 of the gas-liquid switching valve 1 of the present application normally communicates with a gas supply source, the liquid inlet 102 typically communicates with a liquid supply source, and the gas-liquid outlet 103 may communicate with an insertion tube of an endoscope to deliver gas from the gas supply source into a body cavity or into a duct (i.e., in a gas supply state), or to deliver liquid from the liquid supply source into a body cavity or into a duct (i.e., in a liquid supply state).

Notably, as shown in FIGS. 1 to 5, the gas-liquid outlet 103 can serve as a shared outlet in the valve body 10 to serve as a gas outlet when the gas-liquid switching valve 1 is in the gas supply state, and serve as a liquid outlet when the gas-liquid switching valve 1 is in the liquid supply state, helping to simplify the overall structure of the gas-liquid switching valve 1 to facilitate communicating with one gas supply and liquid supply channel in the insertion tube of the endoscope. It will be appreciated that in other examples of the present application, the gas-liquid outlet 103 in the valve body 10 may be two separate outlets located at different positions of the valve body 10, and may still jointly communicate with one gas supply and liquid supply channel in the insertion tube of the endoscope through a tee or the like, which will not be described in detail herein.

Exemplarily, as shown in FIGS. 2 and 3, the valve core assembly 20 may include a valve core shaft 21, a first sealing element 22 configured to unblock and block a gas path between the gas inlet 101 and the gas-liquid outlet 103, and a second sealing element 23 configured to unblock and block a liquid path between the liquid inlet 102 and the gas-liquid outlet 103, and the valve core shaft 21 is provided with a valve core cavity 201 having an opening, and a communication hole 202 located at a side wall of the valve core cavity 201 to jointly form the communication channel 200 of the valve core assembly 20 through the valve core cavity 201 and the communication hole 202. The valve core shaft 21 is axially slidably disposed in the valve cavity 100 of the valve body 10, and the first sealing element 22 and the second sealing element 23 are spacedly disposed on the valve core shaft 21. The gas-liquid outlet 103 in the valve body 10 is located between the gas inlet 101 and the liquid inlet 102 in the axial direction of the valve core shaft 21, and the communication channel 200 of the valve core shaft 21 is configured to communicate the gas inlet 101 with the external environment.

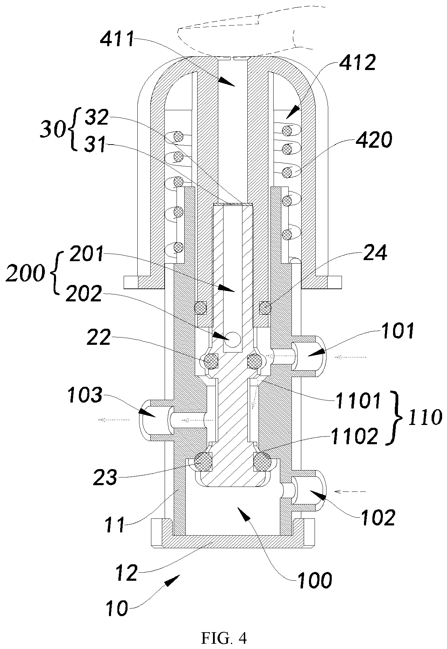

Thus, as shown in FIG. 4, when the valve core shaft 21 is lifted to move to the first position and the communication channel 200 is blocked, a passage between the gas inlet 101 and the external environment is blocked, while the first sealing element 22 no longer blocks the gas path between the gas inlet 101 and the gas-liquid outlet 103, so that gas flowing in via the gas inlet 101 enters the gas-liquid outlet 103 to achieve gas supply; and at this time, the second sealing element 23 blocks the liquid path between the liquid inlet 102 and the gas-liquid outlet 103, so that liquid flowing in via the liquid inlet 102 does not enter the gas-liquid outlet 103 to stop liquid supply.

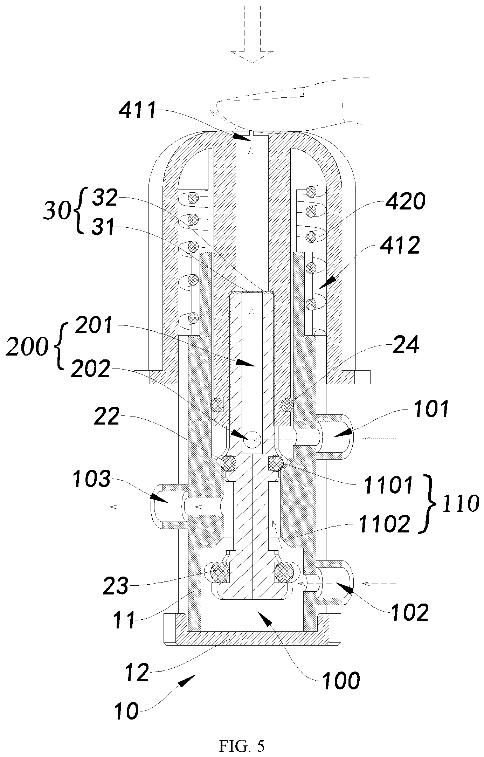

As shown in FIG. 5, when the valve core shaft 21 is pressed to move to the second position, the first sealing element 22 blocks the gas path between the gas inlet 101 and the gas-liquid outlet 103, so that the gas flowing in via the gas inlet 101 does not enter the gas-liquid outlet 103 but is discharged into the external environment through the communication channel 200; and at this time, the second sealing element 23 no longer blocks the liquid path between the liquid inlet 102 and the gas-liquid outlet 103, so that the liquid flowing in via the liquid inlet 102 enters the gas-liquid outlet 103 to achieve liquid supply.

Preferably, the liquid-proof and gas-permeable member 30 is correspondingly disposed at the opening of the valve core cavity 201 of the valve core shaft 21.

Optionally, as shown in FIGS. 2 and 3, both the first sealing element 22 and the second sealing element 23 are implemented as sealing rings sleeving the valve core shaft 21.

Optionally, as shown in FIGS. 3 to 5, the valve cavity 100 of the valve body 10 is provided with a first sealing face 1101 and a second sealing face 1102, wherein the first sealing face 1101 is located between the gas inlet 101 and the gas-liquid outlet 103, and the second sealing face 1102 is located between the liquid inlet 102 and the gas-liquid outlet 103, and the first sealing face 1101 and the second sealing face 1102 sequentially partition the valve cavity 100 into a gas inlet cavity corresponding to the gas inlet 101, an outlet cavity corresponding to the gas-liquid outlet 103, and a liquid inlet cavity corresponding to the liquid inlet 102 along the axial direction of the valve core shaft 21.

Thus, as shown in FIG. 4, when the gas-liquid switching valve 1 is in the gas supply state, the second sealing element 23 airtightly abuts against the second sealing face 1102 to block a gap between the valve core shaft 21 and the second sealing face 1102, so that the liquid path between the liquid inlet 102 and the gas-liquid outlet 103 is blocked; and at this time, the first sealing element 22 leaves the first sealing face 1101 to open a gap between the valve core shaft 21 and the first sealing face 1101, so that the gas path between the gas inlet 101 and the gas-liquid outlet 103 is unblocked.

Whereas, as shown in FIG. 5, when the gas-liquid switching valve 1 is in the liquid supply state, the second sealing element 23 leaves the second sealing face 1102 to open the gap between the valve core shaft 21 and the second sealing face 1102, so that the liquid path between the liquid inlet 102 and the gas-liquid outlet 103 is unblocked; and at this time, the first sealing element 22 airtightly abuts against the first sealing face 1101 to block the gap between the valve core shaft 21 and the first sealing face 1101, so that the gas path between the gas inlet 101 and the gas-liquid outlet 103 is blocked.

Optionally, the valve body 10 includes an annular sealing platform 110 protruding from a side wall of the valve cavity 100 to respectively provide the first sealing face 1101 and the second sealing face 1102 by upper and lower end surfaces of the annular sealing platform 110, so that the outlet cavity of the valve cavity 100 forms a thin section of the valve cavity 100, the liquid inlet cavity and the gas inlet cavity of the valve cavity 100 form two thick sections of the valve cavity 100, that is, the thin section of the valve cavity 100 is located between the two thick sections of the valve cavity 100, so as to provide the first sealing face 1101 matched with the first sealing element 22 by the upper end surface of the annular sealing platform 110, and provide the second sealing face 1102 matched with the second sealing element 23 by the lower end surface of the annular sealing platform 110, so that the first sealing element 22 can be in surface contact with the first sealing face 1101, and the second sealing element 23 can be in surface contact with the second sealing face 1102, contributing to improve the airtight performance during blocking.

Notably, in the present application, an outer diameter dimension of the first sealing element 22 may be smaller than an inner diameter dimension of the gas inlet cavity of the valve cavity 100, and is greater than an inner diameter dimension of the outlet cavity of the valve cavity 100; and an outer diameter dimension of the second sealing element 23 may be smaller than an inner diameter dimension of the liquid inlet cavity of the valve cavity 100, and is greater than the inner diameter dimension of the outlet cavity of the valve cavity 100.

Preferably, the upper and lower end surfaces of the annular sealing platform 110 extend inwardly obliquely, respectively, to smoothly transition the valve cavity 100 from the thick sections to the thin section, contributing to further improve the sealing airtightness between the first sealing element 22 and the first sealing face 1101, and the sealing airtightness between the second sealing element 23 and the second sealing face 1102. Such an arrangement helps to further reduce the amount of liquid that overflows to the external environment through the communication channel 200. Specifically, as shown in FIG. 5, when the gas-liquid switching valve 1 is controlled to switch from the liquid supply state to the gas supply state, the valve core shaft 21 slides upwards, causing part of the liquid to stay in the outlet cavity, and normally, the unblocking of the gas path may cause the liquid staying in the outlet cavity to overflow to the external environment. However, in the technical solution of the present application, in addition to blocking the liquid by the liquid-proof and gas-permeable member 30, the following structure is also advantageous to reduce the amount of the liquid that overflows. More specifically, when an operator releases a control member 41, the gas-liquid switching valve 1 sequentially generates the following actions: a. the valve core shaft 21 slides upwards to the first position, and the control member 41 stops moving; and b. a finger leaves the control member 41, and a through hole 411 is unblocked. In other words, when the finger still blocks the through hole 411, a gas path has been established between the gas inlet 101 and the gas-liquid outlet 103 (i.e. a gas pressure is formed in the gas path), at this time, the inner diameter dimension of the outlet cavity is smaller than the inner diameter dimension of the gas inlet cavity, so that the liquid staying in the outlet cavity is either pushed by the gas to be discharged from the gas-liquid outlet 103 or pressed into a space between the gas-liquid outlet 103 and the second sealing element 23, thereby reducing liquid that overflows along the gas inlet cavity and the communication channel 200. In addition, a design that the first sealing face 1101 is inclined inward makes a gas path gradually open, further strengthening the above beneficial effects.

Notably, as shown in FIG. 5, when the valve core shaft 21 of the valve core assembly 20 is pressed to move to the second position, the gas path between the gas inlet 101 and the gas-liquid outlet 103 is blocked, and at this time, gas from the gas inlet 101 does not enter the gas-liquid outlet 103 regardless of whether the communication channel 200 of the valve core shaft 21 is blocked or opened; while the liquid path between the liquid inlet 102 and the gas-liquid outlet 103 is unblocked, liquid from the liquid inlet 102 will enter the gas-liquid outlet 103. In other words, when the valve core shaft 21 of the valve core assembly 20 is pressed, the gas-liquid switching valve 1 is in a liquid supply state regardless of whether the communication channel 200 of the valve core shaft 21 is blocked or not, i.e., only liquid is supplied and no gas is supplied at this time.

Similarly, as shown in FIGS. 3 and 4, when the valve core shaft 21 of the valve core assembly 20 is lifted to move to the first position, the liquid path between the liquid inlet 102 and the gas-liquid outlet 103 is blocked, and liquid from the liquid inlet 102 will not enter the gas-liquid outlet 103; and the gas path between the gas inlet 101 and the gas-liquid outlet 103 is unblocked. At this time, as shown in FIG. 4, if the communication channel 200 of the valve core shaft 21 is blocked so that a gas path between the gas inlet 101 and the external environment is blocked, gas from the gas inlet 101 will enter the gas-liquid outlet 103 so that the gas-liquid switching valve 1 is in a gas supply state, i.e., only gas is supplied and no liquid is supplied at this time.

However, as shown in FIG. 3, if the communication channel 200 of the valve core shaft 21 is opened when the valve core shaft 21 is lifted to move to the first position, so that the gas path between the gas inlet 101 and the external environment is unblocked, the gas from the gas inlet 101 will preferentially pass through the communication channel 200 to be discharged into the external environment since there is a certain internal pressure in the body cavity or duct communicating with the gas inlet 101, which is generally higher than the atmospheric pressure of the external environment, so that the gas-liquid switching valve 1 is supplied with neither liquid nor gas. At the same time, since the gas path between the gas inlet 101 and the gas-liquid outlet 103 remains unblocked, the gas from the gas inlet 101 can still ensure a certain positive pressure at the gas-liquid outlet 103, so as to maintain the internal pressure of the body cavity or duct, and prevent liquid or gas in the body cavity or duct from flowing back, causing pressure loss in the body cavity or duct.

According to the above embodiment of the present application, as shown in FIGS. 1 and 2, the gas-liquid switching valve 1 may further include a reset mechanism 40, wherein a resetting mechanism 40 is disposed on the valve body 10, and is configured to apply a reset force to the valve core shaft 21 so that the valve core shaft 21 is automatically lifted to return to the first position after a pressing force is removed, so that the liquid path between the liquid inlet 102 and the gas-liquid outlet 103 is blocked, and the gas path between the gas inlet 101 and the gas-liquid outlet 103 is unblocked.

Exemplarily, as shown in FIGS. 2 and 3, the reset mechanism 40 of the gas-liquid switching valve 1 may include a control member 41 and an elastic member 42, wherein the control member 41 is fixedly connected to the valve core shaft 21, and is partially exposed outside the valve cavity 100 of the valve body 10, wherein the elastic member 42 is correspondingly disposed between the control member 41 and the valve body 10, and is configured to apply an elastic force away from the valve body 10 to the control member 41.

Thus, as shown in FIG. 5, when a pressing force is applied to the control member 41 to move the control member 41 toward a direction close to the valve body 10, the control member 41 drives the valve core shaft 21 to slide inside the valve cavity 100 of the valve body 10 so that the first sealing element 22 is close to and in airtight contact with the first sealing face 1101 to block the gas path between the gas inlet 101 and the gas-liquid outlet 103; and at this time, the second sealing element 23 is away from the second sealing face 1102 to unblock the liquid path between the liquid inlet 102 and the gas-liquid outlet 103, so that the state of the gas-liquid switching valve 1 is switched to the liquid supply state.

Whereas, as shown in FIG. 3, if the pressing force applied to the control member 41 is removed, i.e., the control member 41 is released, the control member 41 will move toward a direction away from the valve body 10 under the action of the elastic member 42 to drive the valve core shaft 21 to reversely slide inside the valve cavity 100 of the valve body 10, so that the first sealing element 22 is away from the first sealing face 1101 to unblock the gas path between the gas inlet 101 and the gas-liquid outlet 103; and at this time, the second sealing element 23 is close to and in airtight contact with the second sealing face 1102 to block the liquid path between the liquid inlet 102 and the gas-liquid outlet 103, so that the state of the gas-liquid switching valve 1 is automatically restored to a natural state.

In other words, as shown in FIG. 3, when the control member 41 is not pressed and the communication channel 200 is not blocked, the gas-liquid switching valve 1 is in a natural state, i.e., the gas-liquid switching valve 1 supplies neither gas nor liquid; and as shown in FIG. 4, the gas-liquid switching valve 1 is in the gas supply state only when the control member 41 is not pressed and the communication channel 200 is blocked. As shown in FIG. 5, when the control member 41 is pressed, the gas-liquid switching valve 1 is in the liquid supply state regardless of whether the communication channel 200 is blocked or not, facilitating a user to perform corresponding operations according to different needs, conforming to the user's operation habits, and reducing the risk of misoperation.

Optionally, as shown in FIGS. 2 and 3, the control member 41 of the reset mechanism 40 may be, but is not limited to be, implemented as a button 410. The elastic member 42 of the reset mechanism 40 may be, but is not limited to be, implemented as a spring 420.

Preferably, as shown in FIGS. 3 to 5, the button 410 is detachably connected to the valve core shaft 21, and one end of the spring 420 abuts against the button 410, and the other end of the spring 420 abuts against the valve body 10. Thus, when the button 410 is pressed, the spring 420 is elastically deformed to apply a reaction force to the button 410, so that the button 410 is driven by the elastic force of the spring 420 to return to the original position after being released. It can be appreciated that when the button 410 is pressed, the spring 420 is implemented as a compression spring if the spring 420 is compressed; and the spring 420 is implemented as a tension spring if the spring 420 is stretched. In addition, the spring 420 of the present application may be located outside the valve cavity 100 of the valve body 10 to prevent the spring 420 from contacting liquid to be corroded or contaminate the liquid; of course, in other examples of the present application, the spring 420 may also be located in the valve cavity 100 of the valve body 10, and one end of the spring 420 abuts against the valve core shaft 21, and the other end of the spring 420 abuts against the valve body 10, and the button 410 can be automatically restored to the original position by applying a reaction force to the valve core shaft 21, which will not be described in detail herein.

Optionally, as shown in FIGS. 2 and 3, the valve core shaft 21 of the valve core assembly 20 may have a sealing end 211, and a control end 212 extending axially from the sealing end 211, wherein the first sealing element 22 and the second sealing element 23 spacedly sleeve the sealing end 211 of the valve core shaft 21, and the communication channel 200 is disposed at the control end 212 of the valve core shaft 21.

Correspondingly, as shown in FIGS. 3 to 5, the button 410 is provided with a through hole 411, and an annular cavity 412 surrounding the through hole 411. The control end 212 of the valve core shaft 21 is fixedly inserted into the through hole 411 of the button 410, and the through hole 411 communicates the opening of the valve core cavity 201 in the valve core shaft 21 with the external environment to enable communication of the communication channel 200 with the external environment. At this time, the user can block the communication channel 200 by simply blocking the through hole 411 of the button 410 to achieve the gas supply operation.

Optionally, the spring 420 is sleeved with the annular cavity 412 of the button 410 to not only hide the spring 420 to prevent the spring 420 from being damaged, but also to guide the deformation of the spring 420 to prevent the spring 420 from being twisted and deformed so as not to provide a desired reset force.

Preferably, as shown in FIG. 2, the through hole 411 of the button 410 is provided with convex threads 413 to increase a pressing force between the button 410 and the control end 212 of the valve core shaft 21, so that the valve core shaft 21 can be stably inserted into the button 410, preventing the valve core shaft 21 and the button 410 from being unintentionally or accidentally detached. It can be understood that the convex threads 413 of the present application may be, but are not limited to be, implemented as striplike bumps or the like protruding inward from an inner wall of the through hole 411, which will not be described in detail herein. In addition, although the convex threads 413 can increase the stability of the connection between the button 410 and the valve core shaft 21, the button 410 can be detached from the valve core shaft 21 when a large external force is applied, facilitating the disassembly and assembly of the gas-liquid switching valve 1.

More preferably, as shown in FIGS. 2 and 3, the valve core assembly 20 further includes a third sealing element 24, wherein the third sealing element 24 is located between the button 410 and the valve body 10 to hermetically block a gap between the button 410 and the valve body 10, so that the valve cavity 100 of the valve body 10 communicates with the external environment only through the opening of the valve core cavity 201 in the valve core shaft 21. It will be appreciated that the third sealing element 24 of the present application may also be, but is not limited to be, implemented as a sealing ring that sleeves an inner peripheral wall of the annular cavity 412 of the button 410; and of course, in other examples of the present application, the third sealing element 24 may also directly sleeve the valve core shaft 21 to correspondingly block a gap between the valve core shaft 21 and the valve body 10, still ensuring that the valve cavity 100 of the valve body 10 communicates with the external environment only through the opening of the valve core cavity 201 in the valve core shaft 21, which will not be described in detail herein.

According to the above embodiment of the present application, as shown in FIGS. 2 and 3, the sealing end 211 of the valve core shaft 21 may be provided with a first mounting groove 2111 close to the control end 212, and a second mounting groove 2112 away from the control end 212, wherein the first sealing element 22 is embedded into the first mounting groove 2111 at the sealing end 211, and the second sealing element 23 is embedded into the second mounting groove 2112 at the sealing end 211, so as to ensure that the first sealing element 22 and the second sealing element 23 securely sleeve the valve core shaft 21, respectively.

Preferably, a maximum radial dimension of the first mounting groove 2111 at the sealing end 211 is smaller than an inner diameter dimension at the outlet cavity of the valve cavity 100 of the valve body 10, and a maximum radial dimension of the second mounting groove 2112 at the sealing end 211 is greater than the inner diameter dimension of the outlet cavity of the valve cavity 100 of the valve body 10. At the same time, an outer diameter dimension of the control end 212 of the valve core shaft 21 is also smaller than the inner diameter dimension of the outlet cavity of the valve cavity 100 of the valve body 10. In this way, the control end 212 of the valve core shaft 21 and the first mounting groove 2111 at the sealing end 211 can pass through the outlet cavity of the valve cavity 100 while the second mounting groove 2112 at the sealing end 211 of the valve core shaft 21 cannot pass through the outlet cavity of the valve cavity 100, at this time, after the control member 41 of the reset mechanism 40 is correspondingly mounted at the control end 212 of the valve core shaft 21, the valve core shaft 21 is stably held in the valve cavity 100 of the valve body 10, so that the valve core shaft 21 is prevented from being detached from the valve cavity 100 under the action of the elastic member 42. It will be appreciated that this design also has the advantage that when the valve core assembly 20 is in the first position, liquid enters the liquid inlet cavity of the valve cavity 100 from the liquid inlet 102, which facilitates the formation of pressure on a support platform 2113 or the sealing end 211 in various embodiments, facilitating the sealing of the second sealing element 23 to be more reliable.

It should be noted that each of the above mounting grooves includes all the parts forming the mounting groove, i.e., including a groove bottom, a groove wall, and an extension forming the groove wall.

Optionally, as shown in FIG. 2, the sealing end 211 of the valve core shaft 21 may further be provided with a support platform 2113 for supporting the second sealing element 23, and a radial dimension of the support platform 2113 is greater than an inner diameter dimension of the annular sealing platform 110.

Notably, as shown in FIGS. 1 to 5, in order to facilitate disassembly and assembly and maintenance of the gas-liquid switching valve 1, the valve body 10 of the present application may include a valve cylinder body 11 and a valve cover body 12, wherein the valve cylinder body 11 has a hollow structure penetrating from top to bottom, and the valve cover body 12 is detachably mounted at a lower end of the valve cylinder body 11 to hermetically block an opening at the lower end of the valve cylinder body 11, thereby j ointly forming the valve cavity 100 of the valve body 10 through the valve cylinder body 11 and the valve cover body 12. At the same time, the control member 41 of the reset mechanism 40 is correspondingly disposed at an upper end of the valve cylinder body 11. It will be appreciated that the gas inlet 101, the liquid inlet 102 and the gas-liquid outlet 103 of the valve body 10 are all formed in a peripheral wall of the valve cylinder body 11.

Thus, when the gas-liquid switching valve 1 is assembled, first, after the first sealing element 22 and the second sealing element 23 correspondingly sleeve the sealing end 211 of the valve core shaft 21, respectively, the control end 212 of the valve core shaft 21 is penetrated into the valve cylinder body 11 from the opening at the lower end of the valve cylinder body 11 from the bottom up; then, after the elastic member 42 and the third sealing element 24 are correspondingly mounted on the control member 41, the control member 41 is pressed in from an opening at the upper end of the valve cylinder body 11 to be fixedly connected to the control end 212 of the valve core shaft 21; and finally, the valve cover body 12 is mounted at the lower end of the valve cylinder body 11 to sealingly block the opening at the lower end of the valve cylinder body 11, thereby completing the assembly of the gas-liquid switching valve 1. It will be appreciated that when it is desired to disassemble the gas-liquid switching valve 1, the valve core shaft 21 can be taken out only by pulling the control member 41 out from the upper end of the valve cylinder body 11, and removing the valve cover body 12 from the lower end of the valve cylinder body 11, which is convenient for maintenance or cleaning.

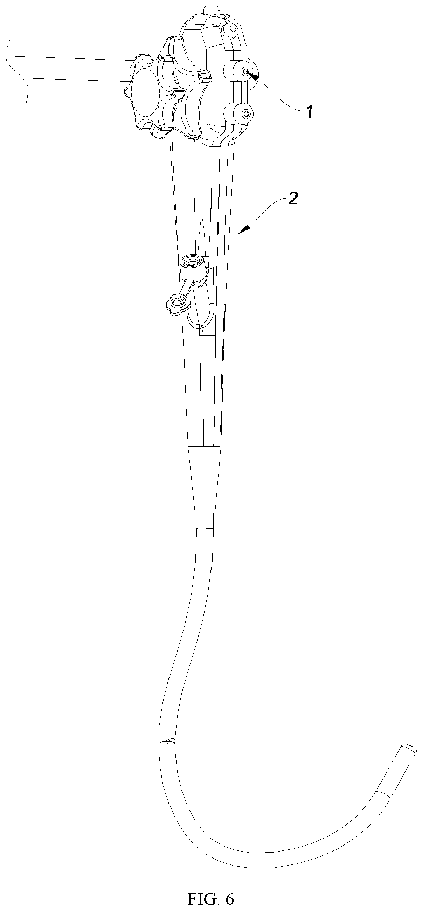

According to another aspect of the present application, as shown in FIG. 6, an embodiment of the present application further provides an endoscope, wherein the endoscope includes an endoscope body 2 and the above gas-liquid switching valve 1, and the gas-liquid switching valve 1 is correspondingly disposed in an operation portion of the endoscope body 2 for switching gas and liquid supplied to an insertion section of the endoscope body 2, thereby realizing the feeding of water, liquid medicine, air, or the like into a body cavity as required. It can be understood that the specific structure and operation principle of the endoscope body 2 of the present application can refer to the endoscope structure in the prior art, and the structure and operation principle of the gas-liquid switching valve 1 of the present application have been described in the above description of the embodiments of the gas-liquid switching valve, which will not be described in detail herein.

The technical features of the above embodiments can be combined without changing the basic principle of the present application, and all possible combinations of the technical features in the above embodiments are not described for the sake of brevity of description, however, as long as there is no contradiction in the combinations of the technical features, they should be regarded as falling within the scope of the description.

The above embodiments are merely illustrative of a few implementations of the present application, and are described in a more specific and detailed manner, but are not therefore to be construed as limiting the scope of the present application. It should be noted that for those of ordinary skill in the art, several variations and modifications can be made without departing from the concept of the present application, which fall within the scope of protection of the present application. Therefore, the protection scope of the present application should be subject to the appended claims.

## Claims

1. A gas-liquid switching valve, **characterized by** comprising:
a valve body provided with a valve cavity, a gas inlet, a liquid inlet, and a gas-liquid outlet, wherein the gas inlet, the liquid inlet and the gas-liquid outlet all communicate with the valve cavity;
a valve core assembly, wherein the valve core assembly is movably disposed in the valve cavity to move between a first position and a second position, and the valve core assembly is provided with a communication channel for communicating the valve cavity with an external environment, wherein when the valve core assembly is moved to the first position, a liquid path between the liquid inlet and the gas-liquid outlet is blocked by the valve core assembly, and a gas path between the gas inlet and the gas-liquid outlet is unblocked, and at this time, the communication channel is blocked so that the gas-liquid switching valve is in a gas supply state; wherein when the valve core assembly is moved to the second position, a gas path between the gas inlet and the gas-liquid outlet is blocked by the valve core assembly, and a liquid path between the liquid inlet and the gas-liquid outlet is unblocked, so that the gas-liquid switching valve is in a liquid supply state; and
a liquid-proof and gas-permeable member, wherein the liquid-proof and gas-permeable member is disposed in the communication channel of the valve core assembly, and is configured to prevent liquid from passing through the communication channel of the valve core assembly, and allow gas to pass through the communication channel of the valve core assembly.

2. The gas-liquid switching valve according to claim 1, **characterized in that** the liquid-proof and gas-permeable member comprises a waterproof gas-permeable membrane, and the waterproof gas-permeable membrane is disposed at an outlet of the communication channel of the valve core assembly.

3. The gas-liquid switching valve according to claim 2, **characterized in that** the liquid-proof and gas-permeable member further comprises a hollow substrate, wherein the hollow substrate is fixed to the valve core assembly, and the waterproof gas-permeable membrane is supportively attached to the hollow substrate.

4. The gas-liquid switching valve according to any one of claims 1 to 3, **characterized in that** the valve core assembly comprises a valve core shaft, a first sealing element configured to unblock and block a gas path between the gas inlet and the gas-liquid outlet, and a second sealing element configured to unblock and block a liquid path between the liquid inlet and the gas-liquid outlet, and the valve core shaft is provided with a valve core cavity having an opening, and a communication hole located at a side wall of the valve core cavity to form the communication channel of the valve core assembly through the valve core cavity and the communication hole, wherein the valve core shaft is axially slidably disposed in the valve cavity of the valve body, and the first sealing element and the second sealing element are spacedly disposed on the valve core shaft.

5. The gas-liquid switching valve according to claim 4, **characterized in that** the valve cavity of the valve body is provided with a first sealing face located between the gas inlet and the gas-liquid outlet, and a second sealing face located between the liquid inlet and the gas-liquid outlet, wherein the first sealing element airtightly abuts against the first sealing face when the valve core shaft of the valve core assembly is pressed to move to the second position, and the second seal airtightly abuts against the second sealing face when the valve core shaft of the valve core assembly is lifted to move to the first position.

6. The gas-liquid switching valve according to claim 5, **characterized in that** the valve body comprises an annular sealing platform protruding from a side wall of the valve cavity to respectively provide the first sealing face and the second sealing face by upper and lower end surfaces of the annular sealing platform.

7. The gas-liquid switching valve according to claim 6, **characterized by** further comprising a reset mechanism, wherein the reset mechanism is disposed on the valve body, and is configured to apply a reset force to the valve core shaft so that the valve core shaft is automatically lifted to return to the first position after a pressing force is removed.

8. The gas-liquid switching valve according to claim 7, **characterized in that** the reset mechanism comprises a control member and an elastic member, wherein the control member is fixedly connected to the valve core shaft, and is partially exposed outside the valve cavity of the valve body, wherein the elastic member is correspondingly disposed between the control member and the valve body, and is configured to apply an elastic force away from the valve body to the control member.

9. The gas-liquid switching valve according to claim 8, **characterized in that** the control member is a button, and the elastic member is a spring, wherein the button is detachably connected to the valve core shaft, and one end of the spring abuts against the button, and the other end of the spring abuts against the valve body.

10. The gas-liquid switching valve according to claim 9, **characterized in that** the valve core shaft has a sealing end, and a control end extending axially from the sealing end, wherein the first sealing element and the second sealing element spacedly sleeve the sealing end of the valve core shaft, and the valve core cavity is located at the control end of the valve core shaft, wherein a through hole, and an annular cavity surrounding the through hole are formed in the button, the control end of the valve core shaft is fixedly inserted into the through hole of the button for communicating the opening of the valve core cavity with an external environment of the valve cavity through the through hole, and the spring is sleeved with the annular cavity of the button.

11. The gas-liquid switching valve according to claim 10, **characterized in that** the through hole of the button is provided with convex threads.

12. The gas-liquid switching valve according to claim 10, **characterized in that** the valve core assembly further comprises a third sealing element, wherein the third sealing element is located between the button and the valve body to hermetically block a gap between the button and the valve body.

13. The gas-liquid switching valve according to claim 10, **characterized in that** the sealing end of the valve core shaft is provided with a first mounting groove close to the control end, and a second mounting groove away from the control end, and the first sealing element is embedded into the first mounting groove, and the second sealing element is embedded into the second mounting groove, wherein an inner diameter dimension of the valve cavity of the valve body at an outlet cavity is greater than a maximum radial dimension of the first mounting groove at the sealing end, and is less than a maximum radial dimension of the second mounting groove at the sealing end.

14. The gas-liquid switching valve according to claim 13, **characterized in that** the sealing end of the valve core shaft is further provided with a support platform for supporting the second sealing element, and a radial dimension of the support platform is greater than an inner diameter dimension of the annular sealing platform.

15. The gas-liquid switching valve according to claim 8, **characterized in that** the valve body comprises a valve cylinder body having a hollow structure penetrating from top to bottom, and a valve cover body, wherein the valve cover body is detachably mounted at a lower end of the valve cylinder body, and the control member of the reset mechanism is correspondingly disposed at an upper end of the valve cylinder body.

16. An endoscope, **characterized by** comprising:
an endoscope body; and
the gas-liquid switching valve according to any one of claims 1 to 15, wherein the gas-liquid switching valve is correspondingly disposed in an operation portion of the endoscope body for switching gas and liquid supplied to an insertion portion of the endoscope body.
